# EUROPÄISCHE PATENTANMELDUNG

(11) **EP 0 911 050 A2**
(43) Veröffentlichungstag der Anmeldung: **28.04.1999**
(21) Anmeldenummer: 98119601.7
(22) Anmeldetag: 16.10.1998
(51) Int. Cl.: A61M 16/08

(54) **Leitungselement für den Anschluss mindestens einer Atemgasleitung für einen Patienten**

(30) Priorität: 16.10.1997 DE 29718137 U; 10.12.1997 DE 29721766 U
(71) Anmelder: Scheu, Rolf Rainer, 60488 Frankfurt/Main (DE)
(72) Erfinder: Scheu, Rolf Rainer, 60488 Frankfurt/Main (DE)
(74) Vertreter: Müller-Gerbes, Margot, Dipl.-Ing.

(57) **Zusammenfassung**

Die Erfindung betrifft ein Leitungselement für den Anschluß mindestens einer Atemgasleitung für einen Patienten, enthaltend einen Korpus mit einem patientenseitigen Ende und mindestens einem leitungsseitigen Ende für den Anschluß der Atemgasleitungen und mit die Enden miteinander verbindenden, durchgängig in dem Korpus ausgebildeten und die Atemgaswege bildenden Lumen, wobei der Atemgasweg jedes Lumens durch ein in das Lumen eingesetztes Einwegeventil in einer Freigaberichtung freigebbar und in der hierzu entgegengesetzten Sperrichtung verschließbar ist, und das Einwegeventil bei einem Vordruck von mindestens 0,005 bis 0,02 bar öffnet und einen Atemgasweg freigibt.

## Beschreibung

Die Erfindung betrifft ein Leitungselement für den Anschluß mindestens einer Atemgasleitung für einen Patienten, enthaltend einen Korpus mit einem patientenseitigen Ende und mindestens einem leitungsseitigen Ende für den Anschluß der Atemgasleitungen und mit die Enden miteinander verbindenden, durchgängig in dem Korpus ausgebildeten und die Atemgaswege bildenden Lumen.

Derartige Leitungselemente werden in vielfältigen medizinischen Anwendungen etwa bei der Atemgasversorgung bzw. Beatmung oder auch der Atemgasanalyse eines Patienten verwendet.

Je nach Art der durchzuführenden Behandlung bzw. Untersuchung des Patienten ist es wünschenswert, den Atemgasstrom in einer bestimmten Richtung zu steuern, so daß eine Trennung des eingeatmeten Atemgases vom ausgeatmeten Atemgas möglich ist. Beispielsweise kann es bei der Untersuchung des ausgeatmeten sogenannten Expirationsgases wünschenswert sein, zur Verhinderung von Meßwertverfälschungen nur das Ausatmen des Patienten durch den Atemgasweg zuzulassen, um an diesem ausgeatmeten Atemgas entsprechende Messungen vornehmen zu können. Ferner ist es bei der Beatmung bzw. Atemgasversorgung eines Patienten wünschenswert, diesem über die an das Leitungselement angeschlossene Atemgasleitung lediglich das Einatmen zu ermöglichen, ein Ausatmen in der entgegengesetzten Richtung in die Atemgasleitung jedoch zu verhindern.

Die Erfindung hat sich daher die Aufgabe gestellt, ein Leitungselement für den Anschluß mindestens einer Atemgasleitung für einen Patienten vorzuschlagen, welches auf möglichst einfache Weise eine zwangsweise Steuerung des Atemgasstromes in der gewünschten Richtung bewirkt. Des weiteren soll auch ein vielseitig verwendbares und wiederholbar einsetzbares Leitungselement geschaffen werden, das auch mehrfach sterilisierbar ist.

Diese Aufgabe wird erfindungsgemäß mit einem Leitungselement gemäß den Merkmalen des Patentanspruches 1 gelöst.

Vorteilhafte Ausgestaltungen und Weiterbildungen der Erfindung sind in den Unteransprüchen angegeben.

Zur Lösung der gestellten Aufgabe schlägt die Erfindung vor, daß in jedes die Atemgaswege durch das erfindungsgemäße Leitungselement bildende Lumen ein Einwegeventil eingesetzt ist, so daß der Atemgasweg jedes Lumens durch das in das Lumen eingesetzte Einwegeventil in einer Freigaberichtung freigebbar und in der hierzu entgegengesetzten Sperrichtung verschließbar ist, und das Einwegeventil bei einem Vordruck von mindestens 0,005 bis 0,02 bar öffnet und den Atemgasweg bereits freigibt. Die im Rahmen der Erfindung vorgeschlagenen Einwegeventile ermöglichen auf einfache Weise die gewünschte Steuerung des Atemgasstromes durch das Leitungselement, wobei je nach Orientierung des Einwegeventils im Atemgasweg das erfindungsgemäße Leitungselement wahlweise nur zum Ausatmen des Patienten oder - bei in entgegengesetzter Richtung eingesetztem Einwegeventil - nur zum Einatmen des Patienten dient und von daher den Atemgasstrom durch das erfindungsgemäße Leitungselement auf einfache Weise zu steuern vermag. Durch den äußerst geringen Vordruck von lediglich mindestens 0,005 bis 0,02 bar, der als Überdruck in Freigaberichtung zum Öffnen des Einwegeventils und Freigeben des Atemgasweges bereits ausreicht, wird hierbei das Ein- bzw. Ausatmen des Patienten über das Einwegeventil in der jeweiligen Freigaberichtung nahezu nicht behindert und gleichzeitig bereits unter diesen Bedingungen ein maximaler Durchfluß an Atemgas über das Einwegeventil und den hierdurch freigegebenen Atemgasweg ermöglicht.

Das im Rahmen der Erfindung eingesetzte Einwegeventil wird vorteilhaft von einem rohrförmigen Ventilgehäuse und einem in das Ventilgehäuse eingesetzten pilzförmigen Ventilkörper mit Ventilschaft und Ventilschirm gebildet, wobei in dem Ventilgehäuse eine ringförmige Anlagefläche von einem umlaufenden und von der Innenwand des Ventilgehäuses vorkragenden Auflagesteg sowie eine koaxial in dem Ventilgehäuse angeordnete Buchse ausgebildet ist und der Ventilschaft des Ventilkörpers in der Buchse gehaltert ist und der Ventilschirm des Ventilkörpers an der Anlagefläche dichtend anliegt und bei einem Vordruck von 0,005 bis 0,02 bar in Freigaberichtung elastisch von der Anlagefläche abhebt. Bei der in der Atemgasversorgung eines Patienten vorherrschenden geringen Durchflußrate an Atemgas ermöglicht diese Konstruktion mit einem ortsfest innerhalb des Ventilgehäuses angeordneten Ventilkörper, bei dem zur Freigabe des Atemgasweges lediglich der elastische Ventilschirm von der Anlagefläche abhebt, eine stets zuverlässige Funktion des Einwegeventils. Auf diese Weise wird zudem ein Einwegeventil geschaffen, welches mit sehr niedrigen Kosten herstellbar ist und ein zuverlässiges Öffnen in Freigaberichtung schon bei geringsten Vordrücken und auch einen zuverlässigen Verschluß des Atemgasweges in Sperrichtung ermöglicht.

Der Ventilschaft des Ventilkörpers weist vorteilhaft eine Verdickung auf, die vom Ventilschirm in einem der Länge der Buchse entsprechenden Abstand auf dem Ventilschaft angeordnet ist, so daß der Ventilschaft in der Buchse ortsfest zwischen dem Ventilschirm und der Verdickung gehaltert ist. Das im Rahmen der Erfindung vorgeschlagene Einwegeventil für das Leitungselement kommt somit mit Ausnahme des elastisch von der Anlagefläche abhebbaren Ventilschirms ohne bewegliche Teile aus.

Zur Erhöhung der Elastizität ist der Ventilschirm hierbei vorteilhaft möglichst dünnwandig ausgebildet und im Übergangsbereich zum Ventilschaft ausgesteift, was nachfolgend noch näher erläutert wird.

Der Ventilschaft des Ventilkörpers ist bevorzugt auf der der Freigaberichtung abgewandten Seite des Ventilschirmes angeformt.

Eine vorteilhafte Weiterbildung des erfindungsgemäßen Leitungselementes sieht vor, daß der Auflagesteg des Einwegeventiles kuppenförmig zur Ausbildung einer annähernd linienförmigen Anlagefläche im Scheitelbereich ausgebildet ist. Hierdurch wird eine geringstmögliche, nämlich idealisiert linienförmige und ringförmig verlaufende Dichtfläche im Ventilgehäuse geschaffen, auf der der Ventilschirm des den Atemgasweg verschließenden Einwegeventiles aufliegt. Die Dichtfläche verläuft dabei quer, bevorzugt senkrecht zur Längsachse des Ventilkörpers und zur Durchströmrichtung des Einwegeventils. Diese geringstmögliche Auflage- bzw. Dichtfläche des Ventilschirmes auf dem Auflagesteg des Ventilgehäuses erlaubt auch bei erhöhter Adhäsion, zum Beispiel durch Feuchtigkeit, die aus dem Atemgas am Ventilschirm kondensieren kann, ein stets leichtes Öffnen des Einwegeventils. Die annähernd linienförmige Anlagefläche am Auflagesteg kann beispielsweise durch Ausbildung eines Radius auf der dem Ventilschirm zugewandten Seite geschaffen werden, wie auch die Ausbildung eines möglichst spitzwinkligen Querschnittes des Auflagesteges eine annähernd linienförmige Anlagefläche für den Ventilschirm schaffen kann.

Damit das erfindungsgemäße Leitungselement den Anforderungen an Sterilität und Verträglichkeit gerecht wird, wird vorgeschlagen, daß das Einwegeventil aus bis mindestens 130°C temperaturbeständigen Materialien hergestellt ist, wobei der Ventilkörper aus einem thermoplastischen und/oder elastomeren Kunststoff, wie Silikon, und das Ventilgehäuse aus einem demgegenüber härter eingestellten Kunststoff, wie Polycarbonat oder Polysulfon, hergestellt ist. Durch die Temperaturbeständigkeit bis mindestens 130°C wird ermöglicht, das Einwegeventil mittels Dampfsterilisation auf einfache Weise zu sterilisieren und auch mehrfach nach erneuter Dampfsterilisation wieder zu verwenden.

Besonders vorteilhaft ist eine Ausbildung des Ventilkörpers aus einem elastischeren und weicheren Kunststoff als dem des Ventilgehäuses mit der darin angeordneten Buchse, um einerseits ein Abheben des Ventilschirmes von der Anlagefläche bei bereits geringstem Vordruck in Freigaberichtung zu ermöglichen und andererseits auch eine einfache und sichere Montage ohne zusätzliche Befestigungsmittel im Ventilgehäuse bzw. der darin ausgebildeten Buchse zu ermöglichen.

Auch die übrigen Teile des erfindungsgemäßen Leitungselementes, wie Korpus, Kuppluingsring etc. werden vorteilhaft aus einem bis mindestens 130°C temperaturbeständigen und damit mittels Dampfsterilisation sterilisierbaren Material, beispielsweise einem geeigneten thermoplastischen und/oder elastomeren Kunststoff, hergestellt.

Um einen festen Sitz des in das Lumen des Leitungselementes eingesetzten Einwegeventils zu gewährleisten, wird außerdem vorgeschlagen, daß das Ventilgehäuse des Einwegeventils außenseitig leicht konisch ausgebildet ist.

Das im Rahmen der Erfindung vorgeschlagene Leitungselement eignet sich für eine Vielzahl von Anschlußsituationen, bei denen mindestens eine Atemgasleitung mit einem Patienten verbunden werden muß. Beispielsweise kann hierzu das patientenseitige Ende mit einem Mundstück oder einer Maske für den Patienten ausgerüstet werden, um Atemgas über die Atemgasleitung von einer Atemgasquelle zum Patienten heranzuführen bzw. von dem diesem ausgeatmetes Gas, beispielsweise zu einem Atemgasanalysegerät über eine angeschlossene Atemgasleitung abzuführen.

Das am patientenseitigen Ende vorgesehene Mundstück oder die Maske kann entweder als separates Teil am patientenseitigen Ende des erfindungsgemäßen Leitungselementes befestigt werden, ggf. mittels einer zusätzlichen Befestigungsvorrichtung oder integral am patientenseitigen Ende des Korpus des Leitungselementes als Mundstück oder Maske angeformt sein.

Eine vorteilhafte Ausführungsform sieht hierbei vor, daß das patientenseitige Ende mit einem Lippenring als Mundstück ausgebildet ist. Ferner können an dem Lippenring nach innen vorstehende Beißblöcke angeformt sein, um das Mundstück vom Patienten einfach im Mund halten zu lassen.

Um das patientenseitige Ende mit einem separat anzubringenden Mundstück oder einer Maske für den Patienten auszurüsten, kann der Korpus am patientenseitigen Ende außenseitig mit einem Kupplungsring versehen sein, der um seine Mittelachse drehbar ist, und der Kupplungsring entlang seines Außenumfanges eine umlaufende Nut für die Befestigung eines entsprechend ausgebildeten Mundstückes oder einer Maske aufweisen. Durch die um seine Mittelachse ausgebildete Drehbarkeit ermöglicht der Kupplungsring bei in die umlaufende Nut eingesetztem Mundstück oder Maske ein leichtes Justieren der hieran befestigten Maske oder des Mundstückes in die für den Patienten angenehmste Position.

Um einem unerwünschten Entweichen von Atemgas über den auf das patientenseitige Ende des Korpus aufgebrachten Kupplungsring zu verhindern, kann ferner der Korpus am patientenseitigen Ende außenseitig mindestens eine umlaufende Nut zur Aufnahme eines Dichtungsringes für die Abdichtung des patientenseitigen Endes des Korpus gegenüber dem Kupplungsring aufweisen.

Weitere Fixierungsmöglichkeiten des erfindungsgemäßen Leitungselementes am Patienten können dadurch geschaffen werden, daß im Bereich des Außenumfanges eines leitungsseitigen Endes des Korpus mindestens ein Befestigungszapfen für die Befestigung eines Haltebandes ausgebildet ist, welches zum Beispiel um den Kopf des Patienten gelegt wird.

Eine weitere vorteilhafte Ausführungsform der Erfindung sieht vor, daß das mindestens eine Lumen im Korpus zum leitungsseitigen Ende hin stufenförmig unter Ausbildung eines Aufnahmeraumes erweitert ist, in welchen das Einwegeventil eingesetzt ist. Auch ist es möglich, bei anderen Anwendungsfällen das Lumen im Korpus zum patientenseitigen Ende hin stufenförmig unter Ausbildung eines Aufnahmeraumes erweitert auszubilden, in welchen das Einwegeventil eingesetzt ist.

In beiden Fällen ist vorteilhaft der stufenförmig erweiterte Aufnahmeraum durch einen in den Aufnahmeraum hineinragenden Haltewulst abgeteilt, wobei das Einwegeventil in das innere Abteil des Aufnahmeraumes ortfest eingesetzt ist. Ein einmal in den Aufnahmeraum eingesetztes Einwegeventil kann von daher ohne größere Manipulationen nicht mehr aus dem erfindungsgemäßen Leitungselement entfernt werden bzw. aus diesem herausfallen.

Bei bestimmten medizinischen Anwendungsfällen, bei denen sowohl eine Zuführung von Atemgas über eine Atemgasleitung zum patientenseitigen Ende und der beispielsweise hier ausgebildeten Maske oder des Mundstückes benötigt wird, wie auch eine in umgekehrter Richtung vom patientenseitigen Ende her erfolgende Ableitung des ausgeatmeten Atemgases in eine weitere Atemgasleitung erforderlich ist, wird vorgeschlagen, daß das erfindungsgemäße Leitungselement zwei leitungsseitige Enden und ein patientenseitiges Ende aufweist, die über die Atemgaswege bildende Lumen miteinander verbunden sind und in jedem Lumen im Bereich des leitungsseitigen Endes des Korpus ein Einwegeventil angeordnet ist, dergestalt, daß ein Einwegeventil den Atemgasweg in der einen Richtung, ausgehend vom patientenseitigen Ende, und das andere Einwegeventil den Atemgasweg in der entgegengesetzten Richtung zum patientenseitigen Ende freigibt und die Einwegeventile in der jeweils entgegengesetzten Richtung den Atemgasweg verschließen, so daß der Atemgasweg zum Einatmen bzw. Ausatmen des Patienten wechselweise über das eine Einwegeventil bzw. das andere Einwegeventil freigebbar ist. Entsprechende an die jeweiligen leitungsseitigen Enden des erfindungsgemäßen Leitungselementes angeschlossene Atemgasleitungen werden somit zwangsweise beim Einatmen bzw. Ausatmen des Patienten über die Lumen mit dem patientenseitigen Ende des Leitungselementes verbunden, so daß ein Einleiten von über die Atemgasleitung zugeführtem Atemgas zum Patienten wie auch die Ableitung von vom Patienten ausgeatmeten Atemgas in eine entsprechende Atemgasleitung ermöglicht ist.

Bei einer solchen Ausbildung des erfindungsgemäßen Leitungselementes mit einem patientenseitigen und zwei leitungsseitigen Enden können die im Korpus ausgebildeten Lumen entweder nach Art eines T oder Y die leitungsseitigen Enden mit dem patientenseitigen Ende verbinden. Demgemäß kann sowohl ein vom patientenseitigen Ende zu einem leitungsseitigen Ende durchgängig verlaufendes erstes Lumen, in welches ein zum zweiten leitungsseitigen Ende führendes zweites Lumen einmündet, wie auch ein vom patientenseitigen Ende her in den Korpus hineinführendes Lumen, welches sich innerhalb des Korpus in zwei zu den beiden leitungsseitigen Enden führrende Lumen verzweigt, vorgesehen sein.

Um das erfindungsgemäße Leitungselement an in der Beatmungstechnik vorhandene Schlauchsysteme und Geräte anschließbar zu machen, wird vorgeschlagen, daß das mindestens eine leitungsseitige Ende für den Anschluß von genormten Konnektoren einer Atemgasleitung nach ISO 5365/1 ausgebildet ist. Diese genormten Konnektoren finden sich an einer Vielzahl von in der Beatmungstechnik und Atemgasversorgung eingesetzten Gerätschaften und Atemgasleitungen, so daß das erfindungsgemäße Leitungselement problemlos in bereits vorhandene Ausrüstungen integrierbar ist.

Die jeweiligen Abmessungen des Leitungselementes wie auch die Durchmesser der Lumen im Korpus sind vorteilhaft ebenfalls an diese genormten Abmessungen von Atemgasleitungen und Geräten angepaßt.

Der Korpus des erfindungsgemäßen Leitungselementes kann ferner je nach Anwendungsfall geradlinig oder abgewinkelt verlaufend ausgebildet sein.

Die Erfindung wird nachfolgend anhand von Ausführungsbeispielen in der Zeichnung näher erläutert. Es zeigen
- Fig. 1: in teilweise geschnittener Darstellung eine Seitenansicht eines erfindungsgemäßen Leitungselementes mit einer daran befestigbaren Nasenmaske
- Fig. 2, 3: in schematischer Darstellung den Zusammenbau des erfindungsgemäßen Leitungselementes gemaß Fig. 1
- Fig. 4: die Aufsicht auf eine weitere Ausführungsform eines erfindungsgemäßen Leitungselementes
- Fig. 5: das Leitungselement gemäß Fig. 4 im Längsschnitt
- Fig. 6: einen Schnitt durch eine weitere Ausführungsform eines erfindungsgemäßen Leitungselementes
- Fig. 7a: das Leitungselement gemäß Fig. 6 mit einer am patientenseitigen Ende befestigbaren Nasenmaske
- Fig. 7b: das Leitungselement gemäß Fig. 6 mit einem am patientenseitigen Ende befestigbaren Mundstück
- Fig. 8: eine weitere Ausführungsform eines erfindungsgemäßen Leitungselementes
- Fig. 9a: in vergrößerter Darstellung einen Schnitt durch ein in den erfindungsgemäßen Leitungselementen eingesetztes Einwegeventil in Verschlußposition
- Fig. 9b: das Einwegeventil gemäß Fig. 9a in geöffneter Position
- Fig. 10: die Aufsicht auf das Ventilgehäuse des Einwegeventiles gemäß Fig. 9a
- Fig. 11: den Schnitt durch das Ventilgehäuse gemäß Linien V-V in Fig. 10
- Fig. 12: eine Seitenansicht des Ventilkörpers des Einwegeventils gemäß Fig. 9a
- Fig. 13: die Unteransicht des Ventilkörpers in teilweise geschnittener Darstellung entlang der Schnittlinien T-T gemäß Fig. 12
- Fig. 14a-e: in schematisierter Darstellung den Zusammenbau des Einwegeventils gemäß Fig. 9.

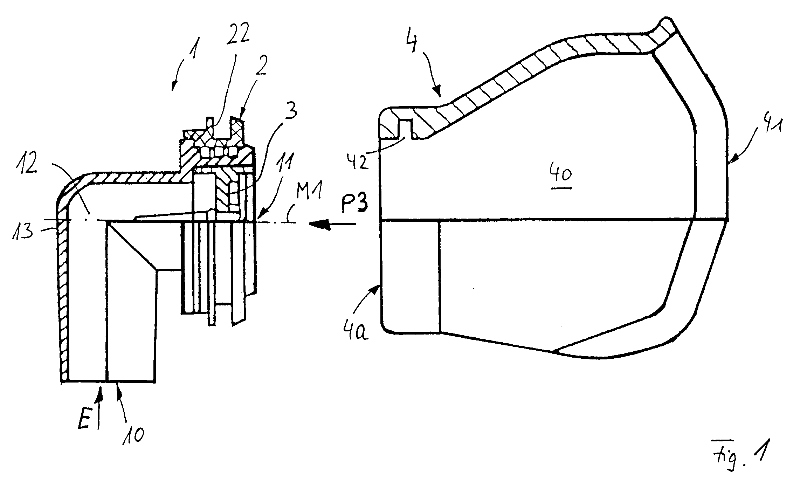

Das in der Fig. 1 dargestellte Leitungselement 1 dient der Atemgasversorgung eines Patienten und weist einen etwa rechtwinklig abgewinkelt verlaufenden rohrförmigen Korpus 13 mit einem Lumen 12 auf, der ein als leitungsseitiges Ende 10 bezeichnetes Ende und ein als patientenseitiges Ende 11 bezeichnetes Ende aufweist. Das leitungsseitige Ende 10 und das patientenseitige Ende 11 sind über das innerhalb des rohrförmigen Korpus 13 durchgängig verlaufende Lumen 12 miteinander verbunden, wobei das Lumen 12 einen Atemgasweg durch das Leitungselement vom leitungsseitigen Ende 10 zum patientenseitigen Ende 11 bildet. Der Korpus 13 des Leitungselementes 1 weist ferner im Bereich des Außenumfanges des patientenseitigen Endes 11 einen auf diesen Außenumfang aufgesteckten Kupplungsring 2 und ein vom patientenseitigen Ende 11 her in das durchgehende Lumen 12 des Leitungselementes 1 eingestecktes Einwegeventil 3 auf, welches nachfolgend noch näher erläutert wird.

Wie bereits eingangs erläutert, dient das in der Fig. 1 dargestellte Leitungselement 1 der Atemgasversorgung eines Patienten. Zu diesem Zweck wird eine hier nicht dargestellte Atemgasleitung, beispielsweise ein Standardbeatmungsschlauch nach DIN/ISO 5356/1 mit dem leitungsseitigen Ende 10 des Leitungselementes 1 verbunden, beispielsweise auf den das durchgehende Lumen 12 umgebenden Korpus 13 vom leitungsseitigen Ende 10 her aufgesteckt, wozu der Korpus 13 in diesem Bereich leicht konisch ausgebildet ist. An dem dem leitungsseitigen Ende 10 abgewandten patientenseitigen Ende 11 des Leitungselementes 1 wird ein Mundstück oder eine Maske beispielsweise die in der Fig. 1 dargestellte Nasenmaske 4 durch Aufstecken gemäß Pfeil P3 angeschlossen, so daß ein durchgehender Atemgasweg über das durchgehende Lumen 12 des Leitungselementes 1 bis in den Innenraum 40 der Nasenmaske 4, ausgehend von einer am leitungsseitigen Ende 10 des Leitungselementes 1 angeschlossenen Atemgasleitung, zur Atemgasversorgung eines Patienten ausgebildet ist. Die Befestigung der Nasenmaske 4 am patientenseitigen Ende 11 des Leitungselementes 1 erfolgt mittels des auf den Korpus 13 im Bereich des patientenseitigen Endes 11 aufgesteckten Kupplungsringes 2, der entlang seines Außenumfanges eine umlaufende Nut 22 aufweist, in die ein auf der Innenseite des Anschlußbereiches 4a der Nasenmaske 4 ausgebildeter Befestigungsbereich 42 einsetzbar ist, wenn die Nasenmaske 4 gemäß Pfeil P3 auf den am patientenseitigen Ende 11 des Korpus 13 angebrachten Kupplungsring 2 aufgesetzt wird.

Um bei der hierdurch ermöglichten Atemgasversorgung eines Patienten den Atemgasstrom durch das Leitungselement 1 zwangsweise in nur einer gewünschten Richtung - hier der Einatemrichtung E des Patienten - zu ermöglichen, ist das Einwegeventil 3 in den vom durchgehenden Lumen 12 des Leitungselementes 1 gebildeten Atemgasweg vom patientenseitigen Ende 11 her eingesetzt. Das Einwegeventil gibt auf Grund seiner konstruktiven Gestaltung lediglich in einer Richtung den Atemgasweg frei und verschließt diesen in entgegengesetzter Richtung, so daß mittels entsprechend orientiertem Einsetzen des Einwegeventils 3 in das durchgehende Lumen 12 die gewünschte zwangsweise Steuerung der Atemgasrichtung durch das Leitungselement 1 bewirkbar ist. In dem in der Fig. 1 dargestellten Ausführungsbeispiel ist das Einwegeventil 3 so in den vom Lumen 12 gebildeten Atemgasweg eingesetzt, daß gemäß Pfeilen E vom leitungsseitigen Ende 10 her Atemgas über die hier angeschlossene Atemgasleitung und das durchgehende Lumen 12 in Einatemrichtung des Patienten in die Nasenmaske 4 gelangen und dort vom Patienten eingeatmet werden kann. In entgegengesetzter Richtung, d.h. der Ausatemrichtung des Patienten, verschließt jedoch das Einwegeventil 3 den vom Lumen 12 ausgebildeten Atemgasweg, so daß in der entgegen Pfeil E gerichteten Richtung kein Atemgas in die Atemgasleitung am leitungsseitigen Ende 10 gelangen kann und eine problemlose Atemgasversorgung des Patienten gewährleistet ist.

Wie insbesondere der Fig. 3 entnehmbar ist, ist das Einwegeventil 3 in der gewünschten Orientierung zur Freigabe bzw. zum Verschließen einer bestimmten Atemgasrichtung, hier zur Freigabe in Einatemrichtung des Patienten gemäß Pfeil E in das durchgehende Lumen 12 des Leitungselementes 1 gemäß Pfeilen P2 vom Patientenseitigen Ende 11 des Korpus 13 her eingesteckt. Zu diesem Zweck weist der das durchgehende Lumen 12 umgebende Korpus 13 zum patientenseitigen Ende 11 hin eine stufenförmige Erweiterung 110 auf, die als Aufnahmeraum für das Einwegeventil 3 dient, so daß dieses gemäß den Pfeilen P2 vom patientenseitigen Ende 11 her in das Leitungselement 1 einsteckbar ist. Um eine sichere Verankerung des in den stufenförmig erweiterten Aufnahmeraum 110 eingesteckten Einwegeventiles 3 zu ermöglichen, kann dieser Aufnahmeraum 110 und die Außenwandung des Ventilgehäuses 30 des Einwegeventiles 3 leicht konisch ausgebildet sein, so daß beim Einstecken des Einwegeventiles 3 in den Aufnahmeraum 110 eine Selbsthemmung zwischen diesen erzeugt wird.

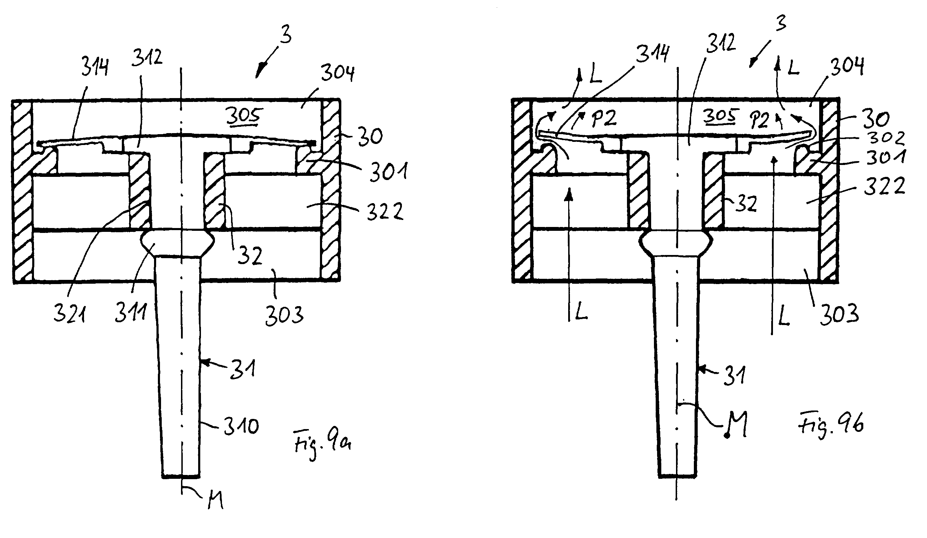

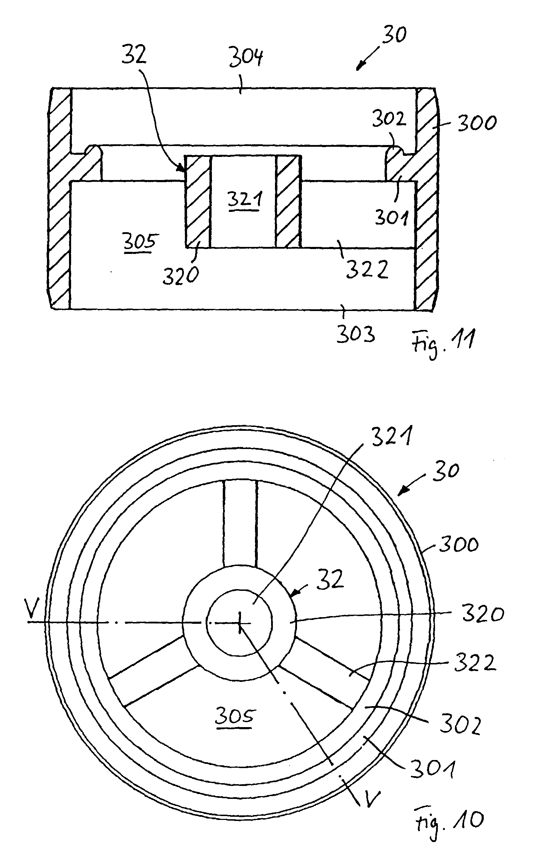

In den Fig. 9a bis 13 ist ein bevorzugter Aufbau eines Einwegeventiles 3 dargestellt. Das Einwegeventil 3 umfaßt ein rohrförmiges Ventilgehäuse 30 mit einem im Inneren ausgebildeten Ventilraum 305 und einen in das Ventilgehäuse 30 eingesetzten pilzförmigen Ventilkörper 31 mit Ventilschaft 310 und Ventilschirm 314. In dem Ventilgehäuse 30 ist, wie sich auch aus den Darstellungen gemäß Fig. 10 und 11 ergibt, eine Buchse 32 mit Wandung 320 und mit einer darin ausgebildeten Durchgangsbohrung 321 ausgebildet, die innerhalb des Ventilraumes 305 koaxial zum Ventilgehäuse 30 gehaltert ist. Hierzu sind, siehe insbesondere Fig. 10, mehrere, hier insgesamt drei sich radial zwischen der Buchse 32 und der Wandung 300 des Ventilgehäuses 30 erstreckende Haltestege 322 vorgesehen, die die Buchse 32 in gewünschten koaxialen Ausrichtung innerhalb des Ventilgehäuses 30 halten. Gleichzeitig ist zwischen den radial den Ventilraum 305 durchsetzenden Haltestegen 322 ein ausreichend großer Freiraum für einen Atemgasdurchgang durch das Einwegeventil 3 gegeben.

Ferner ist in dem Ventilgehäuse 30 eine ringförmige Anlagefläche 302 von einem umlaufenden und von der Innenwand des Ventilgehäuses 30 vorkragenden Auflagesteg 301 ausgebildet. Das Ventilgehäuse 30 mit Auflagesteg 301, Buchse 32 und Haltestegen 322 ist bevorzugt einstückig aus einem thermoplastischen und/oder elastomeren Kunststoff, wie einem Polycarbonat oder Polysulfon, zum Beispiel nach dem Spritzgußverfahren hergestellt.

Das Einwegeventil 3 gemäß Figuren 9a, 9b ist rotationssymmetrisch aufgebaut, d.h. die Mittelachse M ist zugleich die Mittelachse des Ventilkörpers 31, der Buchse 32 und des Ventilgehäuses 30 und stellt auch die Längsachse des Einwegeventiles 3 dar.

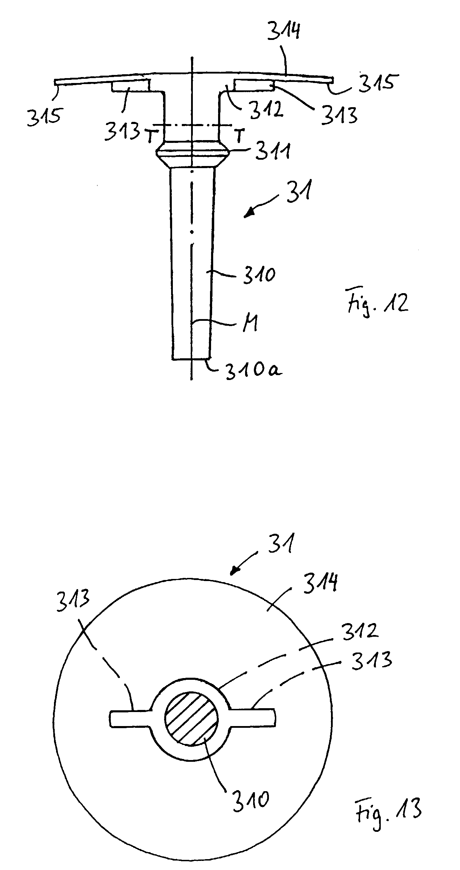

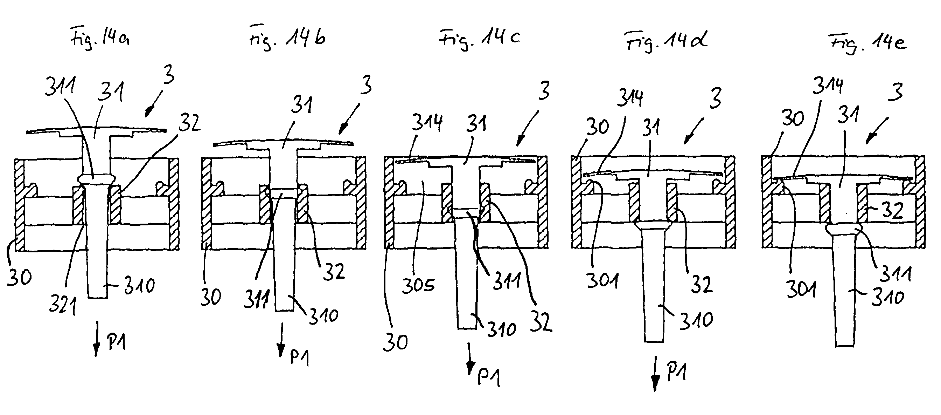

Der pilzförmige Ventilkörper 31, der in näheren Einzelheiten in den Fig. 12 und 13 dargestellt ist, umfaßt einen Ventilschaft 310 mit einem einseitig an den Ventilschaft 310 angeformten und sich quer zur Mittelachse M des Ventilschaftes 310 erstreckenden Ventilschirm 314, so daß der Ventilkörper 31 einen etwa T-förmigen Querschnitt aufweist. Der Ventilschirm 314 ist an seinen äußeren Randbereichen 315 bevorzugt möglichst dünnwandig ausgebildet, ist jedoch im Übergangsbereich 312 zum hieran angeformten Ventilschaft 310 verstärkt ausgebildet. Zu diesem Zweck ist der Übergangsbereich 312 zum einen verdickt ausgebildet. Ferner erstrecken sich beidseits des Ventilschaftes 310 zwei angeformten Stützstege 313 radial nach außen, die mit dem Übergangsbereich 312 und dem Ventilschirm 314 verbunden sind. In der Darstellung gemäß Fig. 12 ist ersichtlich, daß der Ventilschirm von der Mittelachse M ausgehend zu seinen äußeren Randbereichen 315 leicht abwärts geneigt verlaufend, d.h. in Richtung auf das freie Ende 310a des Ventilschaftes 310 verlaufend, ausgebildet ist. Durch die Anordnung der Stützstege 313 auf der dem Ventilschaft 310 zugewandten Seite des Ventilschirmes 314 wird der Ventilschirm 314 in Richtung seines Außenumfanges ausgesteift und kann nur am Außenumfang definierte elastische Bewegungen ausführen.

Zur Ausbildung des in den Fig. 9a, 9b dargestellten Einwegeventiles 3 wird der vorangehend erläuterte Ventilkörper 31 mit seinem Ventilschaft 310 in die Durchgangsbohrung 321 der Buchse 32 innerhalb des Ventilgehäuses 30 eingesteckt. In der in der Fig. 9a, 9b dargestellten Einbauposition ist der Ventilkörper 31 hierbei ortsfest innerhalb der Buchse 32 des Ventilgehäuses 30 gehaltert. Zu diesem Zweck weist der Ventilschaft 310 des Ventilkörpers 31 eine Verdickung 311 auf, die vom Ventilschirm 314 in einem der Länge der Buchse 32 entsprechenden Abstand auf dem Ventilschaft 310 angeordnet ist. Wenn der Ventilkörper 31 in der in den Fig. 9a, 9b dargestellten Endstellung innerhalb der Buchse 32 eingesteckt ist, wird somit eine ortsfeste Verbindung des Ventilkörpers 31 mit der Buchse 32 und damit des Ventilgehäuses 30 geschaffen, wobei der Übergangsbereich 312 des Ventilkörpers 31 an dem einen stirnseitigen Ende der Buchse 32 und die Verdickung 311 an dem anderen stirnseitigen Endbereich der Buchse 32 anliegt und eine weitere Bewegung des Ventilkörpers 31 entlang seiner Mittelachse M nicht mehr möglich ist. Hierbei liegt der Ventilschirm 312 mit seinen in der Fig. 12 mit Bezugsziffer 315 gekennzeichneten Randbereichen auf dem umlaufenden ringförmigen Auflagesteg 301 des Ventilgehäuses 30 abdichtend auf. Die hier ausgebildete Dichtfläche verläuft damit ringförmig umlaufend innerhalb des Ventilgehäuses 30 und ist senkrecht zur Mittelachse M angeordnet.

Das Einstecken des Ventilkörpers 31 in die innerhalb des Ventilgehäuses 30 gelagerte Buchse 32 bis hin zur in den Fig. 9a, 9b dargestellten Einbauposition ist in den Fig. 14a bis 14e in mehreren Schritten dargestellt.

In einem ersten in der Fig. 14a dargestellten Schritt wird das freie Ende 310a des Ventilschaftes 310 des Ventilkörpers 31 in die im Inneren der Buchse 32 ausgebildete Durchgangsbohrung 321 eingeführt und gemäß Pfeil P1 der Ventilkörper 31 durch die Buchse 32 vorgeschoben, bis die Verdickung 311 oberseitig an dem stirnseitigen Ende der Buchse 32 anliegt. Das Einführen des Ventilschaftes 310 in die Buchse 32 wird hierbei durch eine leicht konische Ausbildung des Ventilschaftes 310 zu seinem freien Ende 310a unterstützt. Bei weiterem Vorschieben in Pfeilrichtung P1 wird je nach Elastizitätsverhältnissen der verwendeten Materialien für die Buchse 32 und den Ventilkörper 31 entweder der Ventilkörper 31 unter elastischer Aufweitung der Buchse 32 aufgrund der steiferen Verdickung 311 durch die Buchse 32 hindurchgetrieben oder es erfolgt eine elastische Kompression der Verdickung 311 des Ventilkörpers 31 durch eine demgegenüber steifere Buchse 32, wodurch durch ebenfalls ein weiteres Vorschieben des Ventilkörpers in Pfeilrichtung P1 ermöglicht ist. Bevorzugt ist jedoch der Ventilkörper 31 einstückig aus einem weichen, elastischen Material, z.B. Silikon hergestellt und die Buchse 32 aus einem demgegenüber härter und steifer eingestellten Material gefertigt.

Bei weiterem Vorschieben des Ventilkörpers 31 durch die Buchse 32 taucht, wie in Fig. 14c dargestellt, der Ventilschirm 314 in den vom Ventilgehäuse 30 umgebenden Ventilraum 305 ein und wird, siehe Fig. 14b, an den umlaufenden Anlagesteg 301 des Ventilgehäuses 30 herangeführt. In der in der Fig. 14e dargestellten Endstellung, die auch der Darstellung gemäß Fig. 9a entspricht, liegt der Ventilschirm 314 auf dem nach innen vorkragenden Anlagesteg 301 des Ventilgehäuses 30 auf und der Ventilkörper 31 ist in der Buchse 32 mit seinem Übergangsbereich 312 und der Verdickung 311 in der vorangehend bereits erläuterten Weise festgeklemmt.

In dieser in der Fig. 9a dargestellten Position ist durch die Auflage des Ventilschirmes 314 auf dem Auflagesteg 301 eine Abdichtung gegeben, so daß der Atemgasdurchgangsweg über den Ventilraum 305 innerhalb des Ventilgehäuses 30 verschlossen ist.

Um jedoch in einer gewünschten Richtung ein Öffnen des Einwegeventiles 3 zu ermöglichen, ist der Ventilschirm 314 aus einem elastischen Material, beispielsweise Silikon, hergestellt und im Bereich seines Außenumfanges, mit dem er auch auf dem Anlagesteg 301 aufliegt, möglichst dünnwandig ausgebildet.

Sofern, wie in der Fig. 9b dargestellt, Atemgas gemäß Pfeilen L über die als Gaseintrittsöffnung 303 bezeichnete Öffnung in den Ventilraum 305 eintritt, wirkt auf den Ventilschirm 314 gegenüber der als Gasaustrittsöffnung 304 bezeichneten Seite ein als Vordruck bezeichneter Überdruck ein. Bereits bei einem äußerst geringen Vordruck von beispielsweise 0,01 bis 0,02 bar heben auf Grund dieser Druckverhältnisse die Randbereiche des Ventilschirmes 314 gemäß Pfeilen P2 elastisch vom Auflagesteg 301 des Ventilgehäuses 30 ab, so daß die zuvor ringförmig umlaufende geschlossene Dichtfläche aufgehoben wird und das Atemgas gemäß den Pfeilen L in Fig. 9b um den Außenumfang des Ventilschirmes 314 herum von der Gaseintrittsöffnung 303 zur Gasaustrittsöffnung 304 strömen und dort das Ventilgehäuse 30 wieder verlassen kann. In dieser auch als Freigaberichtung gemäß Pfeilen L bezeichneten Richtung ist von daher auf Grund des bereits für ein Öffnen des Ventiles ausreichenden äußerst geringen Vordruckes ein nahezu ungehinderter Gasdurchgang durch das Einwegeventil 3 ermöglicht.

Hingegen erfolgt in umgekehrter, auch als Sperrichtung bezeichneter Richtung, d.h. entgegen Pfeilen L in Fig. 9b, ein sofortiges Anlegen des Ventilschirmes 314 auf dem Anlagesteg 301 des Ventilgehäuses, so daß von der Gasaustrittsöffnung 304 zur Gaseintrittsöffnung 303 entgegen Pfeilen L kein Atemgas durch das Einwegeventil 3 strömen kann. Diese Sperrwirkung des Einwegeventiles 3 entgegen Pfeil L kann auch durch sehr hohen Atemgasdruck nicht überwunden werden, da dieser lediglich zu einer noch festeren Auflage des Ventilschirmes 314 auf dem Auflagesteg 301 führt, so daß die Dichtwirkung selbsttätig mit dem anliegenden Druck erhöht wird. Bei sehr hohem Druck in Sperrichtung verhindern überdies die Haltestege 322 für die Buchse 32 ein Durchdrücken des Dichtschirmes 314 und damit einen Verlust der Dichtwirkung. Je nach Anforderung können hierzu auich mehr als die dargestellten drei Haltestege 322 vorgesehen sein.

Um auch bei längerem Gebrauch und beispielsweise erhöhter Adhäsion, die sich durch Niederschlag von Feuchtigkeit auf den Ventilschirm 314 aus dem Atemgas ausbilden kann, ein stets leichtes und zuverlässiges Öffnen des Einwegeventiles 3 in der Freigaberichtung zu ermöglichen, ist der Auflagesteg 301 auf seiner der dem Ventilschirm zugewandten Seite kuppenförmig mit einem Radius von z.B. 0,4 mm ausgebildet, wodurch sich eine annähernd linienförmige Anlagefläche 302 im Scheitelbereich für die dichtende Anlage des Ventilschirmes 314 ergibt. Durch diese linienförmige Anlagefläche wird jedoch die der Öffnung des Ventilschirmes 314 unerwünscht entgegenwirkende Adhäsion zwischen Anlagesteg 301 und Ventilschirm 314 auf ein Minimum begrenzt.

Auch der Ventilkörper 31 ist bevorzugt einstückig nach dem Spritzgußverfahren hergestellt, zum Beispiel aus dem bereits erwähnten weichelastischen Kunststoff auf Basis von Silikon.

Sowohl bei der in der Fig. 9a gezeigten abdichtenden Anlage des Ventilschirmes 314 auf dem Anlagesteg 301 des Ventilgehäuses 30 als auch bei dem in der Fig. 9b dargestellten geöffneten Ventilschirm 314 zur Ausbildung eines Atemgasdurchgangsweges durch das Einwegeventil 3 befindet sich der dünnwandige Ventilschirm 314 stets innerhalb des Ventilraumes 305 und ist von der Wandung 300 des Ventilgehäuses 30 umgeben, so daß der empfindliche Ventilschirm 314 im Ventilraum 305 vor Beschädigungen oder Berührung gut geschützt ist.

Mit dem vorangehend erläuterten Einwegeventil 3 ist es somit möglich, bei Einsatz in einem Leitungselement, beispielsweise dem in der Fig. 1 dargestellten Leitungselement 1, den mittels des Lumens 12 ausgebildeten Atemgasweg lediglich in der gewünschten Richtung durch das Leitungselement 1 freizugeben und in der entgegengesetzten Richtung zu verschließen, so daß eine zuverlässige Atemgasversorgung des Patienten gewährleistet ist. Hierzu ist es lediglich erforderlich, daß Einwegeventil 3 in der gewünschten Orientierung in den Aufnahmeraum 110 des Leitungselementes 1 einzusetzen. Bei den in den Figuren dargestellten Ausführungsbeispielen weist hierbei der Ventilschaft 310 des Ventilkörpers 31 mit seinem freien Ende 310a stets entgegen der Freigaberichtung.

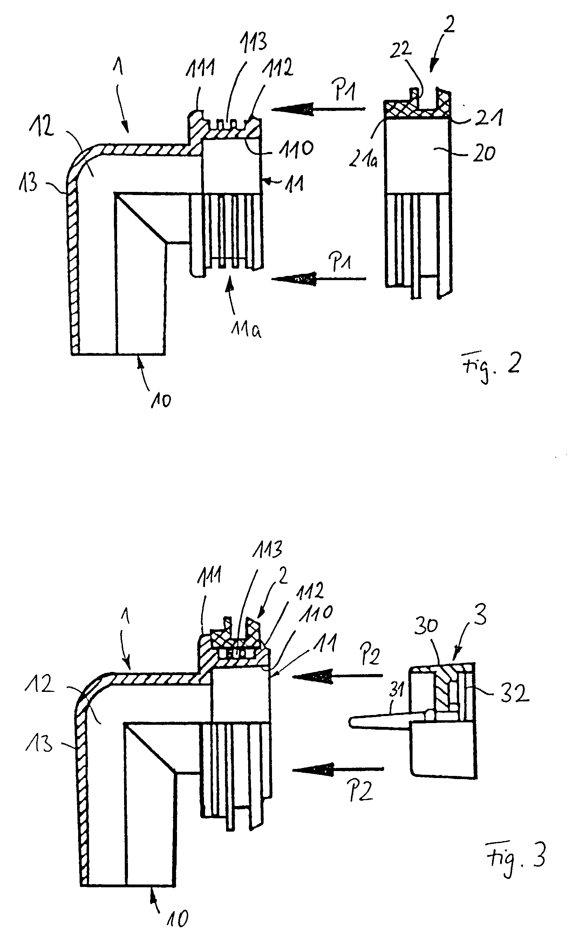

Zur Befestigung der beispielhaft in der Fig. 1 dargestellten Nasenmaske 4 am patientenseitigen Ende 11 des Leitungselementes 1 wird der bereits erwähnte Kupplungsring 2 verwendet, der, wie auch aus Fig. 2 ersichtlich ist, vom patientenseitigen Ende 11 her auf den Korpus 13 des Leitungselementes 1 gemäß Pfeilen P1 aufsteckbar ist. Dazu weist das Leitungselement 1 in seinem an das patientenseitige Ende 11 angrenzenden Bereich einen gegenüber dem rohrförmigen Korpus 13 im Außenumfang vergrößerten Aufsteckbereich 11a auf, der an seinem dem patientenseitigen Ende 11 abgewandten Ende von einem vorstehenden umlaufenden Anschlag 111 und an dem dem patientenseitigen Ende 11 zugewandten Ende von einer umlaufenden Rastnase 112 begrenzt wird. Der Kupplungsring 2, der einen dem Außendurchmesser des Aufsteckbereiches 11a entsprechenden Ringkörper 20 aufweist, ist somit gemäß Pfeilen P1 in Fig. 2 auf das Leitungselement 1 aufsteckbar, und zwar so weit, bis dessen Kante 21a am Anschlag 111 anliegt. In dieser Stellung hinterrastet die umlaufende Rastnase 112 an der gegenüberliegenden Kante 21 des Kupplungsringes 2, so daß dieser gegen ein erneutes Abnehmen vom Leitungselement 1 entgegen Pfeilrichtung P1 gesichert ist und mit dem Korpus 13 des Leitungselementes 1 verbunden wird.

Die Befestigung des Kupplungsringes 2 für die Nasenmaske 4 am Anschlußstück 1 durch Aufstecken und Verrasten in der vorangehend beschriebenen Weise bewirkt darüber hinaus, daß der Kupplungsring 2 zwar gegen ein Abziehen entgegen Pfeilrichtung P1 in Fig. 2 gesichert ist, jedoch um die mit M1 in Fig. 1 gekennzeichnete Mittelachse des patientenseitigen Endes 11 frei drehbar ist, so daß die am Kupplungsring 2 befestigte Nasenmaske 4 mitsamt des Kupplungsringes 2 relativ zum Leitungselement 1 um die Achse M1 drehbar ist, was die Handhabung wesentlich erleichtert. Um darüber hinaus ein ungewolltes Entweichen von Atemgas zwischen dem Korpus 13 des Leitungselementes 1 und dem Kupplungsring 2 zu verhindern, ist der Korpus 13 im Aufsteckbereich 11a mit mehreren, hier drei parallel zueinander verlaufenden Nuten 113 versehen, in die hier nicht dargestellte Dichtungsringe zur Abdichtung des Spaltes zwischen dem patientenseitigen Ende 11 des Korpus 13 des Leitungselementes 1 und dem Kupplungsring 2 eingesetzt werden können.

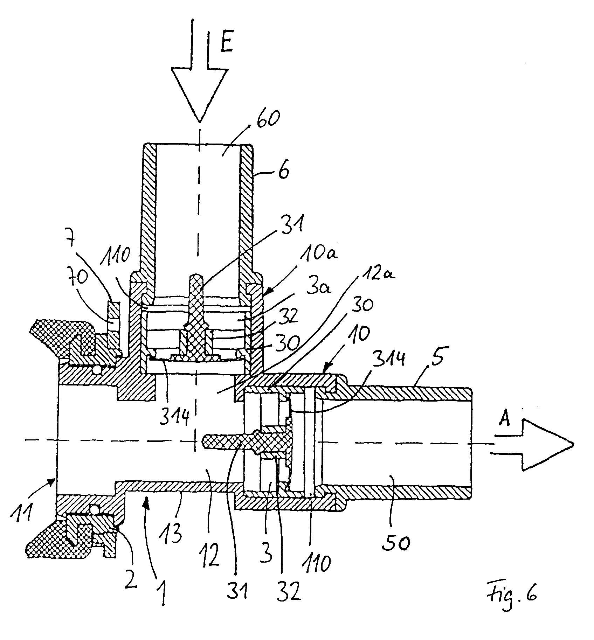

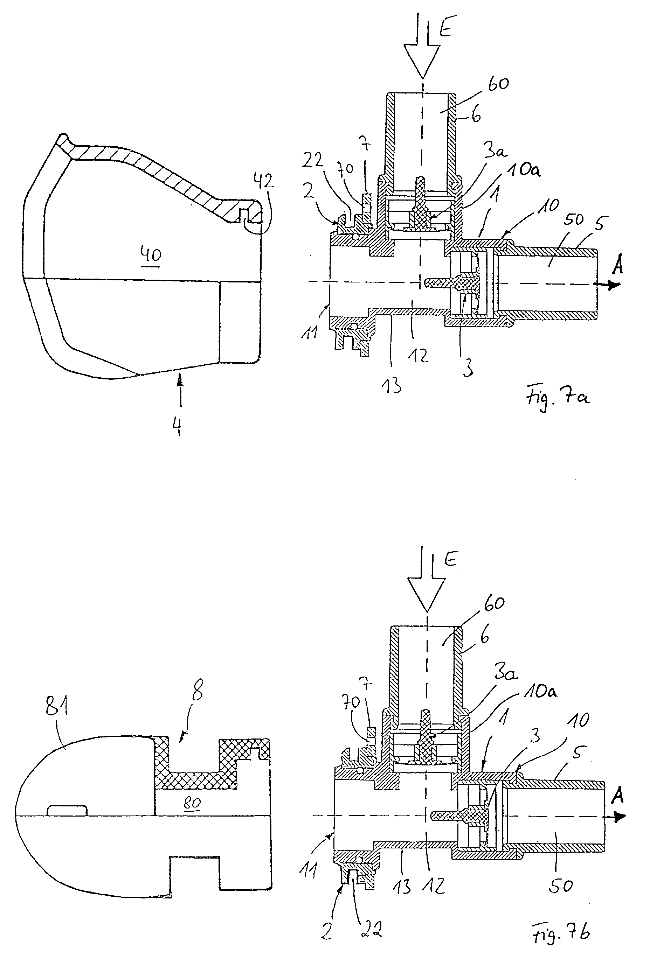

Eine weitere vorteilhafte Ausführungsform des Leitungselementes 1 ist in den Fig. 6, 7a, 7b dargestellt. Das in in diesen Figuren dargestellte Leitungselement weist wiederum einen Korpus 13 mit einem patientenseitigen Ende 11 auf, welches mit einem Kupplungsring 2 in der bereits beschriebenen Weise versehen ist. Im Gegensatz zu dem in der Fig. 1 dargestellten Ausführungsbeispiel weist jedoch der Korpus 13 des Leitungselementes 1 gemäß Fig. 6, 7a, 7b insgesamt zwei leitungsseitige Enden 10, 10a auf, die etwa rechtwinklig zueinander angeordnet sind und über zwei Lumen 12, 12a nach Art eines T miteinander und mit dem patientenseitigen Ende 11 kommunizieren. Hierbei verläuft das Lumen 12 geradlinig durchgängig vom patientenseitigen Ende 11 zum leitungsseitigen Ende 10 des Korpus 13 und das zum leitungsseitigen Ende 10a führende Lumen 12a mündet etwa im mittleren Bereich des Lumens 12 rechtwinklig in dieses ein. In jedes Lumen 12, 12a ist ein Einwegeventil 3 des bereits beschriebenen Aufbaus eingeschoben, hier jedoch vom jeweiligen leitungsseitigen Ende 10, 10a her in einen entsprechenden stufenförmig erweiterten Aufnahmeraum 110 des Korpus 13 eingebracht. Beide leitungsseitigen Enden 10, 10a des Korpus 13 sind im wesentlichen gleich ausgebildet, so daß gleich aufgebaute Einwegeventile 3 in die die Atemgaswege bildenden Lumen 12, 12a eingesetzt werden können.

Auf Grund der Ausbildung des Leitungselementes 1 mit einem patientenseitigen Ende 11, welches mit zwei leitungsseitigen Enden 10, 10a über Lumen 12, 12a verbunden ist, weist das Leitungselement 1 zwei Atemgaswege auf, die von den Lumen 12, 12a gebildet werden. An jedes leitungsseitige Ende 10, 10a können Atemgasleitungen angeschlossen werden. Hierzu sind die mit Bezugszeichen 5 bzw. 6 gekennzeichneten Anschlußkonen nach DIN/ISO 5356/1 an den leitungsseitigen Enden 10, 10a befestigt, beispielsweise mittels der dargestellten einrastbaren und selbsthaftenden Nut- und Federverbindungen. Die Anschlußkonen 5, 6 werden hierbei um ihre Mittelachse drehbar am Leitungselement 1 im Bereich der leitungsseitigen Enden 10, 10a befestigt. Auf diese Anschlußkonen 5, 6 sind sodann in bekannter Weise entsprechende Atemgasschläuche (nicht dargestellt) als Atemgasleitungen aufsteckbar.

Es ist von daher möglich, beispielsweise über den Anschlußkonus 6 am leitungsseitigen Ende 10a Atemgas zum Einatmen durch den Patienten über das patientenseitige Ende 11 zuzuführen, was mit Pfeil E angedeutet ist. Ebenso kann vom Patienten ausgeatmetes Atemgas vom patientenseitigen Ende 11 her über den Atemgasweg durch das Leitungselement 1 in eine beispielsweise am leitungsseitigen Ende 10 auf den Anschlußkonus 5 aufgesteckte Atemgasleitung gemäß Pfeil A abgeleitet werden. Um bei dieser Aufteilung der Atemgaswege stets die gewünschte zwangsweise Steuerung von eingeatmetem Atemgas und ausgeatmetem Atemgas gemäß Pfeilen E, A zu gewährleisten, sind die Einwegeventile 3, 3a in den Lumen 12, 12a in der entsprechenden Orientierung eingesetzt, so daß sie eine zwangsweise Steuerung des Atemgasstromes durch das Leitungselement 1 in der vorangehend geschilderten Weise ermöglichen. Das Einwegeventil 3a im Lumen 12a des leitungsseitigen Endes 10a öffnet lediglich in Einatemrichtung des Patienten gemäß Pfeil E, so daß über das leitungsseitige Ende 10a von der dort angeschlossenen Atemgasleitung zugeführtes Atemgas über das Lumen 12a und einen Teilabschnitt des Lumens 12 zum patientenseitigen Ende 11 strömt und vom Patienten eingeatmet werden kann. In entgegengesetzter Richtung verschließt jedoch das Einwegeventil 3a den Atemgasweg durch das Lumen 12a, so daß vom Patienten in Ausatemrichtung gemäß Pfeil A ausgeatmetes Atemgas nicht in die an das leitungsseitige Ende 10a angeschlossene Atemgasleitung gelangen kann. In dieser Richtung öffnet jedoch das in umgekehrter Orientierung zum Einwegeventil 3a in das Lumen 12 am leitungsseitigen Ende 10 des Korpus 13 eingesetzte Einwegeventil 3, so daß ausgeatmetes Atemgas vom patientenseitigen Ende 11 gemäß Pfeil A durch das Lumen 12 und das Einwegeventil 3 in die an das leitungsseitige Ende angeschlossene Atemgasleitung abgeführt werden kann.

Mit einem derart ausgebildeten Leitungselement 1 mit zwei leitungsseitigen Enden 10, 10a zum Anschluß je einer Atemgasleitung kann somit sowohl das Einatmen des Patienten gemäß Pfeil E als auch das Ausatmen gemäß Pfeil A und die dadurch bedingte Zu- bzw. Abführung des jeweiligen Atemgases über das Leitungselement 1 und ein am patientenseitigen Ende 11 angeschlossenes Mundstück oder eine Maske für den Patienten zwangsweise erfolgen.

Die vorangehend erläuterte Anordnung und Orientierung der Einwegeventile 3, 3a ist lediglich beispielhaft zu verstehen, es ist auch möglich, je nach Anwendungsfall die umgekehrte Orientierung der Einwegeventile 3, 3a vorzusehen.

An das patientenseitige Ende 11 des Leitungselementes 1 ist mittels des in Fig. 1 bereits erläuterten Kupplungsringes 2 sowohl eine in der Fig. 7a dargestellte Nasenmaske 4 wie auch ein in der Fig. 7b beispielhaft dargestelltes Mundstück 8 für den Patienten anschließbar. Beide sind hierzu mit entsprechend angepaßten Befestigungsbereichen ausgebildet. Darüber hinaus können am Kupplungsring 2 noch Halteösen 7 mit Durchgangsöffnungen 70 vorgesehen sein, an denen Haltebänder zur Fixierung des Leitungselementes 1 mitsamt des daran befestigten Mundstückes 8 bzw. der Maske 4 am Kopf des Patienten befestigbar sind.

Die vorangehend beschriebenen Leitungselemente 1 mit Korpus 13 und Kupplungsring 2 ist bevorzugt aus einem Hart-Kunststoff, beispielsweise einem Polycarbonat oder Polysulfon, mit einer Temperaturfestigkeit von mindestens 130°C, beispielsweise nach dem Spritzgußverfahren hergestellt. Diese Materialauswahl ermöglicht, ebenso wie auch beim bereits erläuterten Einwegeventil die mehrfache Wiederverwendung des Leitungselementes 1, da es mittels Dampf mehrfach sterilisierbar ist.

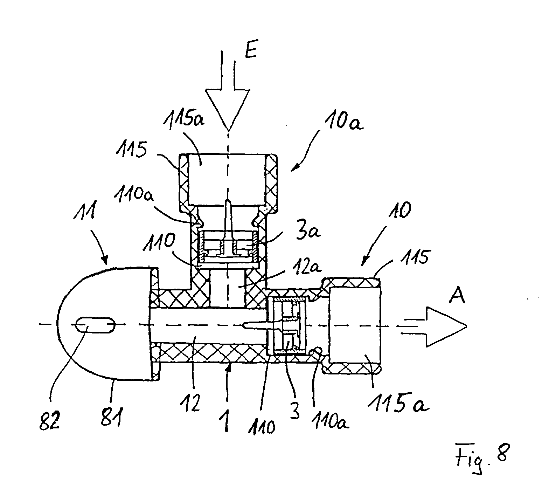

Eine Abwandlung des in der Fig. 7b dargestellten Leitungselementes ist in der Fig. 8 dargestellt. Hierbei ist am patientenseitigen Ende 11 ein Mundstück nicht als separates auf einem Kupplungsring 2 zu befestigendes Teil ausgebildet, sondern an das patientenseitige Ende 11 ist das Mundstück integral angeformt. Auch in diesem Ausführungsbeispiel weist das Leitungselement 1 neben dem als Mundstück ausgebildeten patientenseitigen Ende 11 zwei leitungsseitige Enden 10, 10a auf, die über Lumen 12, 12a, die die Atemgaswege bilden, miteinander verbunden sind. In jedes Lumen 10, 10a ist ein Einwegeventil 3, 3a zur zwangsweisen Steuerung des Einatemgases gemäß Pfeil E und Ausatemgases gemäß Pfeil A eingesetzt. Wiederum sind die Einwegeventil 3, 3a von den leitungsseitigen Enden 10, 10a des Korpus 13 her in entsprechend zum leitungsseitigen Ende hin stufenförmig erweiterte Aufnahmeräume 110 in dem Lumen 12, 12a eingesetzt, wobei die Einwegeventile 3, 3a mittels in den Aufnahmeraum 110 vorstehende Haltewulste 110a in ihrer eingesteckten Position gehalten werden. Die Haltewulste 110a unterteilen den Aufnahmeraum 110, wobei ein Herausfallen des zwischen dem stufenförmigen Absatz und den Haltewulsten 110a im inneren Abteil des Aufnahmeraumes 110 gehalterten Einwegeventils 3 verhindert wird. Ferner sind die leitungsseitigen Enden 10, 10a mit Aufnahmebereichen 115 und entsprechend ausgebildeten Aufnahmebohrungen 115a für den Anschluß genormter Anschlußkonen nach DIN/ISO 5356/1 ausgebildet.

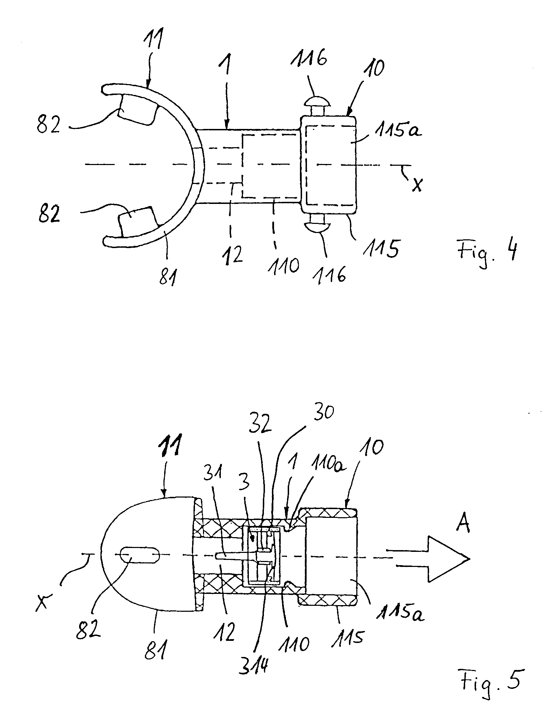

Es ist auch möglich, an Stelle des in der Fig. 8 dargestellten Leitungselementes 1 mit einem patientenseitigen Ende 11 mit angeformtem Mundstück und zwei leitungsseitigen Enden 10, 10a ein Leitungselement gemäß den Fig. 4 und 5 vorzusehen, welches ähnlich wie das Leitungselement gemäß Fig. 1 über nur ein patientenseitiges Ende 11 und ein leitungsseitiges Ende 10 zum Anschluß einer Atemgasleitung verfügt, die über ein durchgehendes Lumen 12 im geradlinig verlaufenden Korpus 13 miteinander verbunden sind. In diesem Fall ist wiederum der Korpus 13 am patientenseitigen Ende 11 mit einem integral angeformten Mundstück ausgebildet, welches von einem Lippenring 81 und nach innen vorstehenden Beißblöcken 82 gebildet ist, wie es auch bei dem in Fig. 8 dargestellten Leitungselement 1 der Fall ist. In den zum leitungsseitigen Ende 10 hin stufenförmig erweiterten Aufnahmeraum 110 des Lumens 12 ist wiederum ein Einwegeventil 3 eingesetzt, welches lediglich ein Ausatmen des Patienten vom patientenseitigen Ende 11 zum leitungsseitigen Ende 10 in eine hieran anschließbare Atemgasleitung ermöglicht, in der entgegengesetzten Richtung jedoch ein Einatmen durch Schließen und Abdichten verhindert. Hierdurch kann beispielsweise eine exakte Analyse des ausgeatmenten Atemgases über ein an die Atemgasleitung angeschlossenes Atemgasanalysegerät vorgenommen werden. Das leitungsseitige Ende 10 ist wiederum für den Anschluß eines genormten Konnektors nach DIN/ISO 5356/1 vorgesehen. Zur Fixierung des Leitungselementes 1 am Patienten sind überdies am leitungsseitigen Ende 10 des Korpus 13 außenseitig vorstehende Befestigungszapfen 116 für die Befestigung eines hier nicht dargestellten Befestigungsbandes vorgesehen, welches beispielsweise um den Kopf des Patienten gelegt werden kann.

## Patentansprüche

1. Leitungselement für den Anschluß mindestens einer Atemgasleitung für einen Patienten, enthaltend einen Korpus mit einem patientenseitigen Ende und mindestens einem leitungsseitigen Ende für den Anschluß der Atemgasleitungen und mit die Enden miteinander verbindenden, durchgängig in dem Korpus ausgebildeten und die Atemgaswege bildenden Lumen, wobei der Atemgasweg jedes Lumens durch ein in das Lumen eingesetztes Einwegeventil in einer Freigaberichtung freigebbar und in der hierzu entgegengesetzten Sperrichtung verschließbar ist, und das Einwegeventil bei einem Vordruck von mindestens 0,005 bis 0,02 bar öffnet und einen Atemgasweg freigibt.

2. Leitungselement nach Anspruch 1,
**dadurch gekennzeichnet**, daß das Einwegeventil von einem rohrförmigen Ventilgehäuse (30) und einem in das Ventilgehäuse (30) eingesetzten pilzförmigen Ventilkörper (31) mit Ventilschaft (310) und Ventilschirm (314) gebildet ist, wobei in dem Ventilgehäuse (30) eine ringförmige Anlagefläche (302) von einem umlaufenden und von der Innenwand des Ventilgehäuses (30) vorkragenden Auflagesteg (301) sowie eine koaxial in dem Ventilgehäuse (30) angeordnete Buchse (32) ausgebildet ist und der Ventilschaft des Ventilkörpers (31) in der Buchse (32) gehaltert ist und der Ventilschirm (314) des Ventilkörpers (31) an der Anlagefläche (302) dichtend anliegt und bei einem Vordruck von mindestens 0,005 bis 0,02 bar in Freigaberichtung elastisch von der Anlagefläche (302) abhebt.

3. Leitungselement nach Anspruch 2,
**dadurch gekennzeichnet**, daß der Ventilschaft (310) des Ventilkörpers (31) eine Verdickung (311) aufweist, die vom Ventilschirm (314) in einem der Länge der Buchse (32) entsprechenden Abstand auf dem Ventilschaft (310) angeordnet ist, so daß der Ventilschaft (310) in der Buchse (32) ortsfest zwischen dem Ventilschirm (314) und der Verdickung (311) gehaltert ist.

4. Leitungselement nach Anspruch 2 oder 3,
**dadurch gekennzeichnet**, daß zur Erhöhung der Elastizität der Ventilschirm (314) dünnwandig ausgebildet und im Übergangsbereich (312) zum Ventilschaft (310) ausgesteift ist.

5. Leitungselement nach einem der Ansprüche 2 bis 4,
**dadurch gekennzeichnet**, daß der Ventilschaft (310) des Ventilkörpers (31) auf der der Freigaberichtung abgewandten Seite des Ventilschirmes (314) angeformt ist.

6. Leitungselement nach einem der Ansprüche 2 bis 5,
**dadurch gekennzeichnet**, daß der Auflagesteg (301) kuppenförmig zur Ausbildung einer annähernd linienförmigen Anlagefläche (302) im Scheitelbereich ausgebildet ist.

7. Leitungselement nach einem der Ansprüche 1 bis 6,
**dadurch gekennzeichnet**, daß das Einwegeventil aus bis mindestens 130°C temperaturbeständigen Materialien hergestellt ist, wobei der Ventilkörper (31) aus einem thermoplastischen und/oder elastomeren Kunststoff, wie Silikon, und das Ventilgehäuse (30) aus einem demgegenüber härter eingestellten Kunststoff, wie Polycarbonat oder Polysulfon, hergestellt ist.

8. Leitungselement nach einem der Ansprüche 1 bis 7,
**dadurch gekennzeichnet**, daß das Ventilgehäuse des Einwegeventils außenseitig leicht konisch ausgebildet ist.

9. Leitungselement nach einem der Ansprüche 1 bis 8,
**dadurch gekennzeichnet**, daß das patientenseitige Ende (11) mit einem Mundstück (8) oder einer Maske (4) für den Patienten ausgerüstet ist.

10. Leitungselement nach Anspruch 9,
**dadurch gekennzeichnet**, daß das Mundstück oder die Maske integral am patientenseitigen Ende (11) angeformt ist.

11. Leitungselement nach einem der Ansprüche 9 oder 10,
**dadurch gekennzeichnet**, daß das patientenseitige Ende (11) mit einem Lippenring (81) als Mundstück ausgebildet ist.

12. Leitungselement nach Anspruch 11,
**dadurch gekennzeichne**t, daß an den Lippenring (81) nach innen vorstehende Beißblöcke (82) angeformt sind.

13. Leitungselement nach einem der Ansprüche 9 bis 12,
**dadurch gekennzeichnet**, daß der Korpus (13) am patientenseitigen Ende (11) außenseitig mit einem Kupplungsring (2) ausgerüstet ist, der um seine Mittelachse drehbar ist und der Kupplungsring entlang seines Außenumfanges eine umlaufende Nut (22) für die Befestigung eines entsprechend ausgebildeten Mundstückes oder einer Maske (4) aufweist.

14. Leitungselement nach Anspruch 13,
**dadurch gekennzeichnet**, daß der Korpus (13) am patientenseitigen Ende (11) außenseitig mindestens eine umlaufende Nut (113) zur Aufnahme eines Dichtungsringes für die Abdichtung des patientenseitigen Endes (11) des Korpus (13) gegenüber dem Kupplungsring (2) aufweist.

15. Leitungselement nach einem der Ansprüche 1 bis 14,
**dadurch gekennzeichnet**, daß im Bereich des Außenumfanges eines leitungsseitigen Endes (10, 10a) des Korpus (13) mindestens ein Befestigungszapfen (116) für die Befestigung eines Haltebandes ausgebildet ist.

16. Leitungselement nach einem der Ansprüche 1 bis 15,
**dadurch gekennzeichnet**, daß das mindestens eine Lumen im Korpus (13) zum leitungsseitigen Ende hin stufenförmig unter Ausbildung eines Aufnahmeraumes (110) erweitert ist, in welchen das Einwegeventil eingesetzt ist.

17. Leitungselement nach einem der Ansprüche 1 bis 15,
**dadurch gekennzeichnet**, daß das Lumen zum patientenseitigen Ende (11) hin stufenförmig unter Ausbildung eines Aufnahmeraumes (110) erweitert ist, in welchen das Einwegeventil eingesetzt ist.

18. Leitungselement nach einem der Ansprüche 16 oder 17,
**dadurch gekennzeichnet**, daß der stufenförmig erweiterte Aufnahmeraum (110) durch einen in den Aufnahmeraum (110) hineinragenden Haltewulst (110a) abgeteilt ist,wobei das Einwegeventil in das innere Abteil des Aufnahmeraumes (110) ortsfest eingesetzt ist.

19. Leitungselement nach einem der Ansprüche 1 bis 18,
**dadurch gekennzeichnet**, daß es zwei leitungsseitige Enden (10,10a) und ein patientenseitiges Ende (11) aufweist, die über die Atemgaswege bildende Lumen (12, 12a) miteinander verbunden sind und in jedem Lumen (12, 12a) im Bereich des leitungsseitigen Endes (10, 10a) ein Einwegeventil (3, 3a) angeordnet ist, dergestalt, daß ein Einwegeventil (3) den Atemgasweg in der einen Richtung, ausgehend vom patientenseitigen Ende (11), und das andere Einwegeventil (3a) den Atemgasweg in der entgegengesetzten Richtung zum patientenseitigen Ende (11) freigibt und die Einwegeventile (3, 3a) in der jeweils entgegengesetzten Richtung den Atemgasweg verschließen, so daß der Atemgasweg zum Einatmen bzw. Ausatmen des Patienten wechselweise über das eine Einwegeventil (3a) bzw. das andere Einwegeventil (3) freigebbar ist.

20. Leitungselement nach einem der Ansprüche 1 bis 19,
**dadurch gekennzeichnet**, daß das mindestens eine leitungsseitige Ende für den Anschluß von genormten Konnektoren einer Atemgasleitung nach DIN/ISO 5365/1 ausgebildet ist.

21. Leitungselement nach einem der Ansprüche 1 bis 20,
**dadurch gekennzeichnet**, daß der Korpus (13) abgewinkelt verlaufend ausgebildet ist.
